# EUROPEAN PATENT APPLICATION

(11) **EP 4 666 973 A1**
(43) Date of publication of application: **24.12.2025**
(21) Application number: 24903009.9
(22) Date of filing: 16.12.2024
(51) Int. Cl.: A61B 18/14, A61B 18/12, A61B 18/08, A61B 10/02, A61B 17/3209

(54) **INCISION APPARATUS FOR ENDOSCOPE**

(30) Priority: 15.12.2023 CN 202311738136
(71) Applicant: Hangzhou AGS MedTech Co., Ltd., Hangzhou, Zhejiang 311103 (CN)
(72) Inventor: HAN, Chunqi, Hangzhou, Zhejiang 311103 (CN); YUAN, Jiabin, Hangzhou, Zhejiang 311103 (CN); ZHENG, Lingyan, Hangzhou, Zhejiang 311103 (CN)
(74) Representative: Wang, Bo
(86) International application number: PCT/CN2024/139488
(87) International publication number: WO 2025/124594

(57) **Abstract**

An endoscopic cutting device is provided. The endoscopic cutting device includes an operating portion, a sheath tube including an injection lumen and a cutting wire lumen, a proximal end of the sheath tube being connected to the operating portion; and a cutting wire at least partially disposed within the cutting wire lumen, a proximal end of the cutting wire being connected to the operating portion. The cutting wire includes a cutting segment extending out of the sheath tube from a sidewall of the sheath tube to form a knife portion for performing a surgical operation.

## Description

### CROSS REFERENCE TO RELATED APPLICATIONS

This application claims priority to Chinese Patent Application No. 202311738136. 8, filed on December 15, 2023, the entire contents of each of which are incorporated herein by reference.

### TECHNICAL FIELD

The present disclosure relates to the field of medical technology, and in particular relates to an endoscopic cutting device.

### BACKGROUND

Endoscopic-sphincterotomy (EST) is a clinical operation utilizing an endoscopic retrograde cholangiopancreatography (ERCP) to cut a duodenal sphincter with a high-frequency electrosurgical knife under endoscopy, thereby enlarging an opening of a bile duct. During the procedure, after completing the ERCP, a cutting wire is gently pulled to bend a front end of a sheath tube into an arc shape, exposing the electrosurgical knife and then a high-frequency electric current is applied to the electrosurgical knife to cut the duodenal papillary sphincter. During this process, if the tension on the cutting wire is not well controlled, the cutting wire is easily broken or disengaged, therefore requiring the operator to have extensive experience.

Therefore, there is a need to provide an endoscopic cutting device to solve the above technical problem.

### SUMMARY

One or more embodiments of the present disclosure provide an endoscopic cutting device including: a sheath tube including an injection lumen and a cutting wire lumen, a proximal end of the sheath tube being connected to an operating portion; a cutting wire at least partially disposed within the cutting wire lumen, the proximal end of the cutting wire being connected to the operating portion. The cutting wire includes a cutting segment, a limiting portion and a rotation segment disposed at different positions of the cutting wire. The cutting segment extends out of the sheath tube from a sidewall of the sheath tube, the limiting portion is disposed within the cutting wire lumen and is configured to form an axial limitation with the cutting wire lumen. The rotation segment is disposed either in the injection lumen or in the cutting wire lumen, and is configured to drive the sheath tube to rotate around an axis of the proximal end of the cutting wire when the operating portion rotates the cutting wire.

In some embodiments, the cutting wire lumen includes a first lumen, and the limiting portion includes a bending segment, the bending segment is disposed on a distal side of the cutting segment, at least a portion of the bending segment is disposed in the first lumen, and the bending segment is configured to drive a distal end of the sheath tube to bend when the operating portion pulls the cutting wire from a distal end to a proximal end of the endoscopic cutting device.

In some embodiments, the limiting portion further includes a first limiting member disposed within the first lumen, the bending segment is fixed to the first limiting member, and the first limiting member is configured to limit an axial displacement of the bending segment relative to the first lumen.

In some embodiments, an engagement structure with an axial limitation is formed between the first limiting member and an inner wall of the first lumen, and the engagement structure includes at least one limiting recess and at least one limiting protrusion extending along a circumferential direction, the at least one limiting recess is disposed on one of an outer wall of the first limiting member and the inner wall of the first lumen, the at least one limiting protrusion is disposed on the other of the outer wall of the first limiting member and the inner wall of the first lumen, and the at least one limiting recess and the at least one limiting protrusion are mutually engaged.

In some embodiments, a shape of a cross-sectional of the first limiting member is circular, and the engagement structure includes one or more of a tower-like structure, a serrated structure, and a threaded structure.

In some embodiments, at least a portion of a cross-sectional shape of the first limiting member is non-circular, and the first limiting member forms a circumferential limitation with the first lumen.

In some embodiments, the first limiting member is constructed as an isotropic cross-sectioned column along an axial direction of the first limiting member, and the first limiting member is fixed in the first lumen through an interference fit.

In some embodiments, a sidewall of the first lumen is formed with a first perforation group and at least one second perforation group communicating with an outer wall of the sheath tube. The at least one second perforation group is disposed at a distal side of the first perforation group, the first perforation group includes a first hole and a second hole, the cutting wire penetrates from the first hole to an outer side of the sheath tube to form the cutting segment, the bending segment penetrates from the second hole to the first lumen, and the rotation segment sequentially penetrates through the at least one second perforation group sequentially and is fixed in the first lumen.

In some embodiments, the endoscopic cutting device further includes a second limiting member axially movably disposed within the first lumen, at least a portion of the rotation segment is fixed to the second limiting member, and the second limiting member is configured to limit a circumferential displacement of the rotation segment relative to the first lumen.

In some embodiments, a circumferentially limiting structure is formed between the second limiting member and an inner wall of the first lumen, the circumferentially limiting structure includes at least one concave portion and at least one convex portion extending along an axial direction, the at least one concave portion is disposed on one of the outer wall of the second limiting member or the inner wall of the first lumen, the at least one convex portion is disposed on the other of the outer wall of the second limiting member and the inner wall of the first lumen, and the at least one concave portion and the at least one convex portion cooperate with each other.

In some embodiments, the cutting wire lumen further includes a second lumen communicating with the first lumen, the second lumen is disposed non-collinearly to the first lumen, and the rotation segment is disposed within the second lumen.

In some embodiments, the second lumen is parallel to the first lumen, the rotation segment extends within the second lumen toward the distal end of the sheath tube or toward the proximal end of the sheath tube.

In some embodiments, an inner diameter of the second lumen is equal to an outer diameter of the rotation segment.

In some embodiments, a first communication hole is formed between the injection lumen and the first lumen, the first communication hole is located at a distal end of the injection lumen, and the rotation segment bends from the first communication hole into the injection lumen, and extends toward the distal end of the sheath tube or toward the proximal end of the sheath tube.

In some embodiments, a diameter of the rotation segment is greater than or equal to an inner diameter of the injection lumen.

In some embodiments, the sheath tube further includes a guide wire lumen, a second communication hole is formed between the guide wire lumen and the injection lumen, and the second communication hole is located between a middle of the sheath tube and the first communication hole, a sealing member is disposed between the first communication hole and the second communication hole to seal the injection lumen.

In some embodiments, the sheath tube includes a tip structure fixed to the distal end of the sheath tube, a diameter of a proximal end of the tip structure is equal to a diameter of the distal end of the sheath tube, and a diameter of the tip structure decreases progressively from the proximal end to a distal end; and the tip structure includes a distal outlet lumen whose proximal end is connected to a distal end of the guide wire lumen.

In some embodiments, the rotation segment is disposed with a developing structure.

In some embodiments, the operating portion includes a pulling portion and a rotating portion. The pulling portion is configured to control the cutting wire to move proximally, the bending segment drives the distal end of the sheath tube to bend, the rotating portion is configured to control the cutting wire to rotate, and the rotation segment drives the sheath tube to rotate; and the rotating portion is connected to a proximal end of the pulling portion; or the rotating portion is spaced apart from the pulling portion.

In some embodiments, the sheath tube further includes a guide wire lumen, a second communication hole is formed between the guide wire lumen and the injection lumen, and the second communication hole is located between a bending origin of the sheath tube and a middle of the sheath tube to direct a solution in the injection lumen to the guide wire lumen, the bending origin being a proximal endpoint of a curved segment of the sheath tube.

In some embodiments, the cutting wire lumen includes a first lumen for accommodating the cutting wire, a first communication hole is formed between the injection lumen and the first lumen, the first communication hole is located at a distal end of the injection lumen, and the rotation segment passes through the first communication hole and is fixed into the injection lumen.

In some embodiments, a diameter of the rotation segment is greater than or equal to an inner diameter of the injection lumen.

In some embodiments, a cross-sectional area of the second communication hole is greater than a cross-sectional area of the injection lumen.

In some embodiments, the injection lumen is disposed with a sealing member disposed between the second communication hole and the distal end of the injection lumen for sealing the injection lumen.

In some embodiments, the cutting wire lumen includes a first lumen accommodating the cutting wire, a first communication hole is formed between the injection lumen and the first lumen, and the rotation segment penetrates through the first communication hole and is fixed in the injection lumen.

In some embodiments, the sheath tube further includes a guide wire lumen, a second communication hole is formed between the guide wire lumen and the injection lumen, and the second communication hole is configured to direct a solution in the injection lumen to the guide wire lumen.

In some embodiments, the first communication hole is located at a distal end of the injection lumen, and the second communication hole and the first communication hole are axially offset along the axial direction of the sheath tube.

According to the solution in the above embodiment, the limiting portion and rotation segment of the cutting wire are separately arranged, whereby the force exerted on the cutting wire when controlling the bending of the sheath tube and the force exerted when controlling the rotation of the sheath tube can be dispersed at different positions of the cutting wire. This prevents stress concentration in the cutting wire during both bending and rotation control of the sheath tube, thereby avoiding the cutting wire breakage or disengagement.

### BRIEF DESCRIPTION OF THE DRAWINGS

The present disclosure will be further illustrated by way of exemplary embodiments, which are described in detail by means of the accompanying drawings. These embodiments are not limiting, and in these embodiments, the same numbering denotes the same structure, wherein:
FIG. 1 is a schematic diagram illustrating an exemplary structure of an endoscopic cutting device according to some embodiments of the present disclosure;
FIG. 2 is a schematic diagram illustrating a structure of a distal end of an endoscopic cutting device according to some embodiments of the present disclosure;
FIG. 3 is a cross-sectional view of a distal end of an endoscopic cutting device according to some embodiments of the present disclosure;
FIG. 4 is a schematic diagram illustrating a distal end of an endoscopic cutting device in a bending state according to some embodiments of the present disclosure;
FIG. 5 is a schematic diagram illustrating a structure of a first limiting member according to some embodiments of the present disclosure;
FIG. 6 is a schematic diagram illustrating a structure of a first limiting member according to some other embodiments of the present disclosure;
FIG. 7 is a cross-sectional view of a distal end of an endoscopic cutting device according to some other embodiments of the present disclosure;
FIG. 8 is a schematic diagram illustrating a cross-section along a line A-A of the endoscopic cutting device according to some embodiments of FIG. 7;
FIG. 9 is a cross-sectional view of a distal end of an endoscopic cutting device according to some other embodiments of the present disclosure;
FIG. 10 is a cross-sectional view illustrating a distal end of an endoscopic cutting device according to some other embodiments of the present disclosure;
FIG. 11A is a partially enlarged view illustrating a region B of the endoscopic cutting device according to some embodiments of FIG. 10;
FIG. 11B is a schematic diagram illustrating a cross-section along a line C-C of the endoscopic cutting device according to some embodiments of FIG. 10;
FIG. 12 is a cross-sectional view of a distal end of an endoscopic cutting device according to some other variant embodiments of the present disclosure;
FIG. 13 is a cross-sectional view illustrating a distal end of an endoscopic cutting device according to some other variant embodiments of the present disclosure;
FIG. 14 is a schematic diagram illustrating a cross-section along a line D-D of the endoscopic cutting device according to some embodiments of FIG. 12;
FIG. 15 is a schematic diagram illustrating a structure of a distal end of an endoscopic cutting device according to some other embodiments of the present disclosure;
FIG. 16 is a partial cross-sectional view of the distal end of the endoscopic cutting device according to FIG. 15;
FIG. 17 is a side view of the distal end of the endoscopic cutting device according to FIG. 15;
FIG. 18A is a schematic diagram illustrating a cross-section along a line E-E of the endoscopic cutting device according to FIG. 17;
FIG. 18B is a schematic diagram of a cross-section along a line F-F of the endoscopic cutting device according to FIG. 17;
FIG. 18C is a schematic diagram illustrating a cross-section along a line G-G of the endoscopic cutting device according to FIG.17;
FIG. 18D is a schematic diagram illustrating a cross-section along a line H-H of the endoscopic cutting device according to FIG. 17;
FIG. 19 is a schematic diagram illustrating a structure of a cutting wire according to some other embodiments of the present disclosure;
FIG. 20A is a partially enlarged view of a region R at the distal end of the endoscopic cutting device according to FIG. 15; and
FIG. 20B is a localized enlarged view of a region R at the distal end of a variant embodiment of the endoscopic cutting device according to FIG.15.

The accompanying drawings are labeled as follows:
100, operating portion; 110, liner assembly frame; 120, metal electrode; 130, guiding catheter; 140, pulling portion; 150, rotating portion; 200, sheath tube; 210, first lumen; 220, first perforation group; 221, first hole; 222, second hole; 230, second perforation group; 231, outlet hole; 232, inlet hole; 240, second lumen; 250, via; 260, injection lumen; 261, first communication hole; 270, guide wire lumen; 271, second communication hole; 272, sealing member; 280, tip structure; 281, distal outlet lumen; 300, cutting wire; 310, cutting segment; 320, bending segment; 330, rotation segment; 400, insulating sleeve; 500, first limiting member; 510, engagement structure; 600, second limiting member; 610, limiting structure.

### DETAILED DESCRIPTION

To more clearly illustrate the technical solutions of the embodiments of the present disclosure, the accompanying drawings required to be used in the description of the embodiments will be briefly described below. Obviously, the accompanying drawings in the following description are only some examples or embodiments of the present disclosure, and it is possible for those skilled in the art to apply the present disclosure to other similar scenarios in accordance with these drawings without creative labor. Unless obviously obtained from the context or the context illustrates otherwise, the same number in the drawings refers to the same structure or operation.

It should be understood that the terms "device," "structure," and/or "module" as used herein is a manner for distinguishing between different components, elements, parts, segments or assemblies at different levels. However, the words may be replaced by other expressions if other words accomplish the same purpose.

As shown in the present disclosure and the claims, unless the context clearly suggests an exception, the words "a," "an," "one" and/or "the" do not refer specifically to the singular, but may also include the plural. Generally, the terms "including" and "comprising" suggest only the inclusion of clearly identified operations and elements that do not constitute an exclusive list, and the method or device may also include other operations or elements.

An endoscopic cutting device may serve as a primary medical instrument for procedures such as endoscopic-sphincterotomy (EST). The endoscopic cutting device includes an operating portion, a sheath tube, a cutting wire, and other components. The operating portion is connected to a proximal end of the sheath tube, the cutting wire extends through an interior of the sheath tube to a distal end of the sheath tube, and a cutting segment is formed on an outer side of the distal end of the sheath tube. By applying a high-frequency electric current to the cutting segment, objects such as the nipple sphincter are cut. During the procedure, an operator gently pulls on cutting wire to bend a front end of the sheath tube, or rotates the cutting wire to rotate the sheath tube. Unexpected incidents like wire fracture or disengagement may occur due to force concentration or connection instability, which elevates surgical risks.

In view of the foregoing, some embodiments of the present disclosure provide an endoscopic cutting device. By spreading out the force applied on the cutting wire at different positions of the cutting wire, for example, by separating a rotation segment of the cutting wire from a bending segment, thereby addressing wire fracture caused by stress concentration. Additionally, through improved fixation manners, for example, by reinforcing the cutting wire through a first limiting member and/or a second limiting member, the endoscopic cutting device prevents wire disengagement under high tension loads.

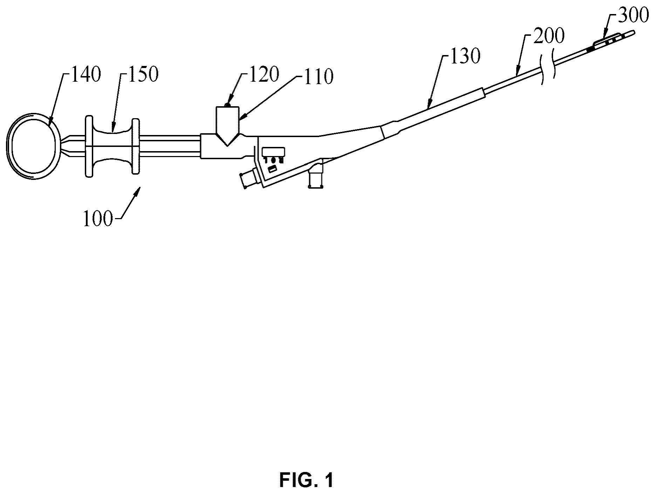

Embodiments of the present disclosure provide an endoscopic cutting device, the endoscopic cutting device aiming to spread the force applied to the cutting wire at different positions of the cutting wire, and to improve a stability of the cutting wire. The exemplary embodiments of Embodiment I of the endoscopic cutting device of the present disclosure is described in detail in the following through FIGs. 1-14.

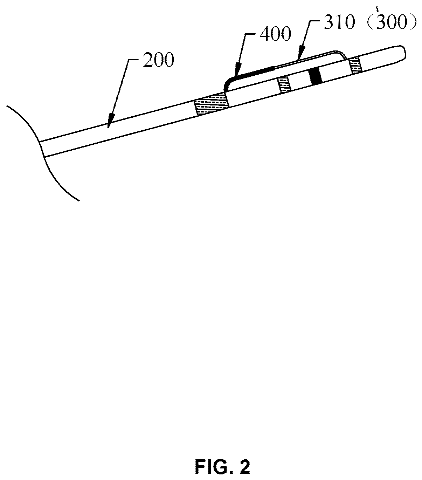

FIG. 1 is a schematic diagram illustrating an exemplary structure of an endoscopic cutting device according to some embodiments of the present disclosure. FIG. 2 is a schematic diagram illustrating a structure of a distal end of an endoscopic cutting device according to some embodiments of the present disclosure.

As shown in FIG. 1 and FIG. 2, the endoscopic cutting device includes an operating portion 100, a sheath tube 200, and a cutting wire 300.

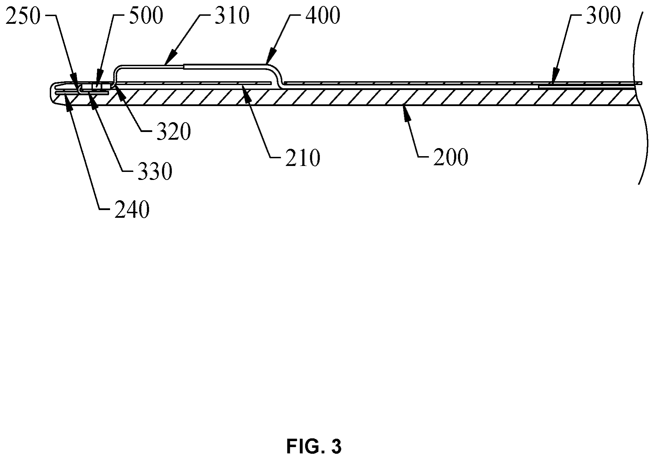

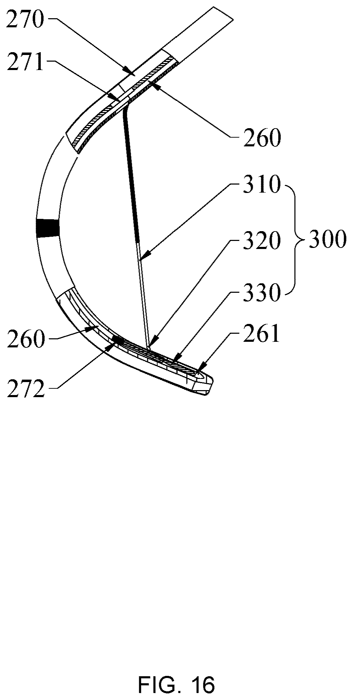

In some embodiments, the sheath tube 200 includes at least one internal lumen, e.g., the sheath tube 200 includes an injection lumen 260 for injecting a solution such as a developer (which is shown in FIG. 16), a guide wire lumen 270 for accommodating a guide wire (as shown in FIG. 16), at least one cutting wire lumen (referred to as a first lumen 210 and a second lumen 240 hereinafter) for accommodating the cutting wire 300 (which is shown in FIG. 3), etc.

In some embodiments, the operating portion 100 is disposed at a proximal end of the sheath tube 200. Exemplarily, the operating portion 100 includes a handle (a hand ring, a hand wheel, etc.) for operating the cutting wire 300, a liner assembly frame 110 connected to a distal end of the handle, a metal electrode 120 disposed on the liner assembly frame 110, and a guiding catheter 130 and other components disposed between the liner assembly frame 110 and the sheath tube 200.

In some embodiments, at least a portion of the cutting wire 300 is accommodated within the sheath tube 200, and a proximal end of the cutting wire 300 is connected to the operating portion 100. The operating portion 100 is available for an operator to hold and control the cutting wire 300, for example, the operating portion 100 controls the cutting wire 300 to move along an axial direction of the sheath tube 200 or rotate around its own axis. The axial direction of the sheath tube 200 refers to a lengthwise direction of the sheath tube 200.

In some embodiments, the cutting wire 300 includes a limiting portion, the limiting portion is disposed within the cutting wire lumen and is configured to form an axial limitation with the cutting wire lumen. Exemplarily, the limiting portion includes a bending segment and a first limiting member 500 (referring to descriptions below), and the limiting portion may be fixed in the cutting wire lumen by the first limiting member 500, or the limiting portion may be disposed in the cutting wire lumen through an interference fit for limiting an axial displacement of the cutting wire 300, so that when the operating portion 100 pulls the cutting wire 300, the cutting wire 300 may drive the sheath tube 200 to bend.

In some embodiments, the cutting wire 300 includes a rotation segment 330, the rotation segment 330 is disposed in the injection lumen 260 or is disposed within the cutting wire lumen and is configured to rotate the sheath tube 200 around the axis of the proximal end of the cutting wire 300 when the operating portion 100 rotates the cutting wire 300. Exemplarily, the rotation segment 330 is disposed within the injection lumen 260. Exemplarily, the cutting wire lumen includes a first lumen 210 and a second lumen 240 (which are described below), and the rotation segment 330 is disposed within the first lumen 210 or within the second lumen 240. By setting the rotation segment 330 independently, it is convenient to spread a torque for driving the sheath tube 200 to rotate by the cutting wire 300 totransmitted by the cutting wire 300 to drive a rotation of the sheath tube 200 to different positions, thereby avoiding a stress concentration.

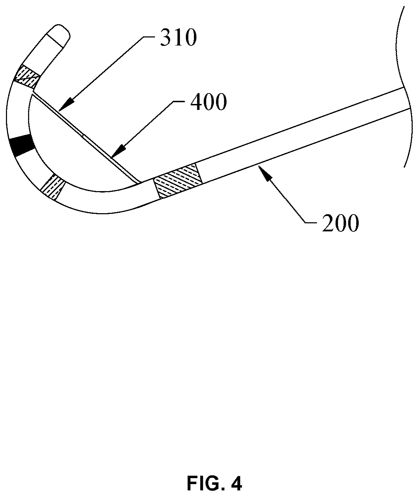

FIG. 3 is a cross-sectional view of a distal end of an endoscopic cutting device according to some embodiments of the present disclosure. FIG. 4 is a schematic diagram illustrating a distal end of an endoscopic cutting device in a bending state according to some embodiments of the present disclosure.

As shown in FIG. 3 and FIG. 4, in some embodiments, the sheath tube 200 includes the first lumen 210 accommodating the cutting wire 300, the first lumen 210 extends from a proximal end of the sheath tube 200 along an axial direction of the sheath tube 200 to a distal end.

In some embodiments, at least a portion of the cutting wire 300 is movably disposed within the first lumen 210 and at least another portion of the cutting wire 300 extends outside of the sheath tube 200 from a sidewall of the sheath tube 200 and is connected to the distal end of the sheath tube 200.

In some embodiments, the limiting portion includes a bending segment 320, i.e., the distal end of the cutting wire 300 includes a cutting segment 310, a bending segment 320, and a rotation segment 330. For example, the cutting segment 310, the limiting portion, and the rotation segment 330 are disposed adjacent to each other on the cutting wire 300. As another example, the cutting segment 310, the limiting portion, and the rotation segment 330 are spaced apart on the cutting wire 300. It is noted that the cutting segment 310, the bending segment 320, and the rotation action segment 330 refer to segments on the cutting wire 300 with corresponding functions, and there is no clear boundary between edges of the segments, e.g., the bending segment 320 refers to an action segment where the cutting wire 300 droves the sheath tube 200 to bend, the rotation segment 330 refers to an action segment where the cutting wire 300 drives the sheath tube 200 to rotate, and small portions at two ends of these segments that do not have a corresponding function may also be divided into corresponding segments.

In some embodiments, the cutting segment 310 includes a segment of the cutting wire 300 disposed outside of the sheath tube 200 for constituting a knife portion that cuts a human tissue. For example, a high-frequency electric current is applied to the cutting segment 310 through the metal electrode 120 on the liner assembly frame 110 to enable the cutting segment 310 to cut a target object (e.g., a nipple sphincter, etc.). In some embodiments, the endoscopic cutting device further includes an insulating sleeve 400, at least a portion of the insulating sleeve 400 is disposed outside of the sheath tube 200. A portion of the cutting segment 310 is disposed within the insulating sleeve 400, and another portion is exposed outside of the insulating sleeve 400 to serve as a knife for cutting. The insulating layer is able to shorten an actual cutting distance of the cutting segment 310 to minimize a trauma. Exemplarily, a portion of the insulating sleeve 400 is disposed within the first lumen 210 of the sheath tube 200, and another portion is disposed on an outer side of the sheath tube 200. Exemplarily, the insulating sleeve 400 is entirely disposed on the outer side of the sheath tube 200. The insulating sleeve 400 is wrapped around the cutting wire 300 and may move with the cutting wire 300. When the operating portion 100 controls an axial movement of the cutting wire 300 relative to the first lumen 210, the insulating sleeve 400 disposed on the outer side of the sheath tube 200 retracts into the first lumen 210.

In some embodiments, the bending segment 320 refers to a portion of the cutting wire 300 that contacts and applies a pulling force on the sheath tube 200 when the distal end of the sheath tube 200 bends. Exemplarily, the bending segment 320 is disposed on a distal side of the cutting segment 310, and the bending segment 320 is configured to drive the distal end of the sheath tube 200 to bend when the operating portion 100 pulls the cutting wire 300 from the distal end to the proximal end. At least a portion of the bending segment 320 is disposed within the first lumen 210, and another portion of the bending segment 320 is exposed from a sidewall of the sheath tube 200. The bending segment 320 is disposed at a position where the distal end of the cutting segment 310 enters the sheath tube 200.

In some embodiments, the cutting wire 300 includes the rotation segment 330, and the rotation segment 330 refers to a portion of the cutting wire 300 that contacts and applies a torque to the sheath tube 200 when the cutting wire 300 rotates about its own axis. In some embodiments, the rotation segment 330 is disposed on a side of the proximal end of the cutting segment 310 and/or the bending segment 320, or on a side of the distal end of the cutting segment 310 and/or the bending segment 320. The rotation segment 330 is configured to drive the sheath tube 200 to rotate around the axis of the proximal end of the cutting wire 300 when the operating portion 100 rotates the cutting wire 300.

In some embodiments, the cutting segment 310, the limiting portion, and the rotation segment 330 are located at different positions of the cutting wire, in this way, by spreading the force applied on the cutting wire at different positions of the cutting wire, a transmission of the force in the various segments is optimized, and a reliability of the cutting wire 300 is improved.

According to the solutions in the above embodiment, the bending segment 320 and the rotation segment 330 of the cutting wire 300 are separately disposed, so that the force applied on the cutting wire 300 when the cutting wire 300 controls the sheath tube 200 to bend and the force applied on the cutting wire 300 when the cutting wire 300 controls the sheath tube 200 to rotate are dispersed at different positions of the cutting wire 300, thereby avoiding an over-concentration of stress on the cutting wire 300 when controlling the bending and the rotation of the sheath tube 200, and preventing the cutting wire 300 from breaking or disengaging.

In the following context, some embodiments of the bending segment 320 and the associated components, such as the first limiting member 500, are described in detail in connection with the accompanying drawings.

In some embodiments, the bending segment 320 forms an axial limitation with the first lumen 210, so that the bending segment 320 drives the distal end of the sheath tube 200 to bend under an action of the pulling force of the operating portion 100 from the distal end to the proximal end. In some embodiments, a manner in which the bending segment 320 forms the axial limitation with the first lumen 210 includes, but is not limited to, disposing the first limiting member 500, interference fit, etc.

In some embodiments, the limiting portion further includes the first limiting member 500 disposed within the first lumen 210. The bending segment 320 is fixed to the first limiting member 500, the first limiting member 500 is configured to limit an axial displacement of the bending segment 320 relative to the first lumen 210. After the axial displacement of the bending segment 320 relative to the first lumen 210 is limited, the operating portion 100 pulls the cutting wire 300 from the distal end to the proximal end so that a proximal end portion of the cutting segment 310, which is located on the outer side of the sheath tube 200, enters the first lumen 210, and the bending segment 320 drives the distal end of the sheath tube 200 to be bent into an arc shape, at which time, the cutting segment 310 changes from a state in contact with the sidewall of the sheath tube 200 to a state spaced apart from the sidewall of the sheath tube 200, thereby facilitating the knife portion to cut the human tissue.

Exemplarily, the bending segment 320 is fixedly connected to the first limiting member 500. Exemplarily, the bending segment 320 is integrally molded with the first limiting member 500.

As shown in FIG. 3, in some embodiments, an engagement structure 510 with an axial limitation is formed between the first limiting member 500 and the inner wall of the first lumen 210. The engagement structure 510 includes at least one limiting recess and at least one limiting protrusion that extends along a circumferential direction. The at least one limiting recess is disposed on one of an outer wall of the first limiting member 500 and the inner wall of the first lumen 210, and the at least one limiting protrusion is disposed on the other of the outer wall of the first limiting member 500 and the inner wall of the first lumen 210. The limiting recess and the limiting protrusion engage with each other. When the limiting recess and the limiting protrusion are engaged, the first limiting member 500 is axially positioned relative to the first lumen 210 so that the bending segment 320 is able to drive the distal end of the sheath tube 200 to bend.

Exemplarily, the engagement structure 510 includes a plurality of limiting protrusions and a plurality of limit recesses. The plurality of limiting protrusions are spaced apart in an axial direction on the inner wall of the first lumen 210 (or the outer wall of the first limiting member 500), and the plurality of limit recesses are spaced apart in the axial direction on the outer wall of the first limiting member 500 (or the inner wall of the first lumen 210), the limiting protrusions and the limit recesses are in a one-to-one correspondence and cooperate with each other.

Exemplarily, the engagement structure 510 includes a plurality of limiting protrusions and a plurality of limiting recesses. At least one of the plurality of limiting protrusions is disposed on the inner wall of the first lumen 210, and the remaining limiting protrusions are disposed on the outer wall of the first limiting member 500. At least one of the plurality of limiting recesses is disposed on the outer wall of the first limiting member 500, and the remaining limiting recesses are disposed in the inner wall of the first lumen 210. In some other embodiments, the limiting protrusion and limiting recess of the engagement structure 510 are of other compositional forms.

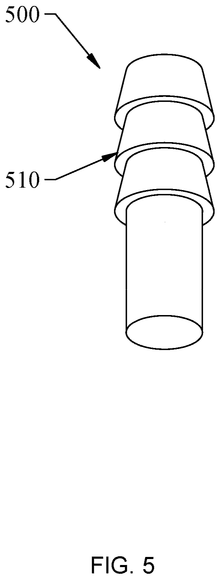

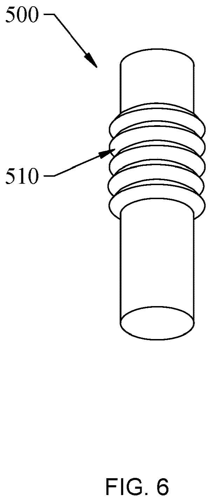

FIG. 5 is a schematic diagram illustrating a structure of a first limiting member 500 according to some embodiments of the present disclosure. FIG. 6 is a schematic diagram illustrating a structure of a first limiting member 500 according to some other embodiments of the present disclosure.

As shown in FIG. 5 and FIG. 6, in some embodiments, the first limiting member 500 limits only an axial displacement of the bending segment 320 relative to the first lumen 210, but does not limit a circumferential displacement of the bending segment 320 relative to the first lumen 210. In some embodiments, the first limiting member 500 has a circular cross-sectional shape, and the first limiting member 500 is capable of rotating relative to the first lumen 210. In some embodiments, the bending segment 320 includes a portion from a position where a distal end of the cutting segment 310 entering the sheath tube 200 to a position where the distal end of the cutting segment 310 is threaded into the first limiting member 500. Due to a degree of freedom of a circumferential movement of the first limiting member 500 relative to the first lumen 210, the rotation segment 330 may be driven to rotate by the first limiting member 500. Exemplarily, when the rotation segment 330 is disposed at the distal end of the bending segment 320 in a surgical operation, the operating portion 100 drives the cutting wire 300 to rotate. The cutting wire 300 drives the first limiting member 500 to rotate, and the first limiting member 500 drives the rotation segment 330 to rotate, so that the rotation segment 330 drives the sheath tube 200 to rotate.

In some embodiments, the first limiting member 500 forms an axial limitation through the engagement structure 510, and the engagement structure 510 includes one or more of a tower-like structure, a serrated structure, and a threaded structure.

Exemplarily, as shown in FIG. 5, the engagement structure 510 includes the tower-like structure constructed from a stack of frustums, each having one larger end and one smaller end, and the end of the tower-like structure with a greater diameter forms an engagement with the inner wall of the first lumen 210.

Exemplarily, the engagement structure 510 includes the serrated structure. The serrated structure is formed by a plurality of rings arranged on the first limiting member 500, as shown in FIG. 6. For example, a cross-section of each of the plurality of rings includes, but is not limited to, a triangle, an arc, a quad, etc.

In some other embodiments, the engagement structure 510 includes other structures such as the threaded structure, etc.

According to the embodiment in the above embodiment, the axial displacement of the bending segment 320 is limited by the first limiting member 500, but the circumferential displacement of the bending segment 320 is not limited, so that the axial displacement and the circumferential displacement are dispersed to prevent the cutting wire 300 from breaking and disengaging.

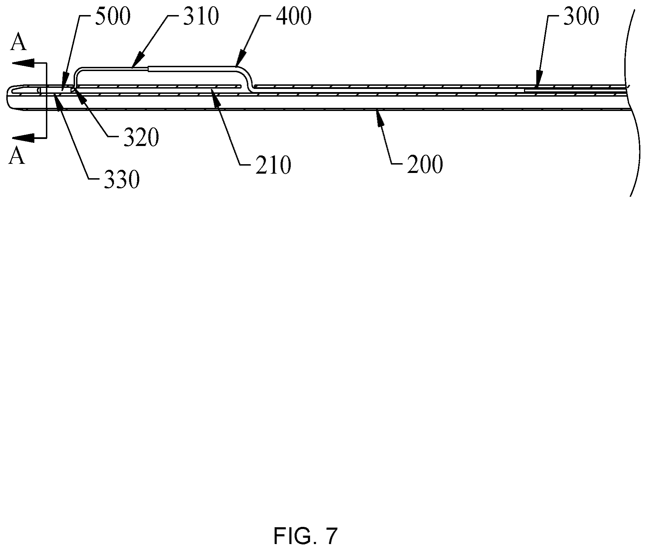

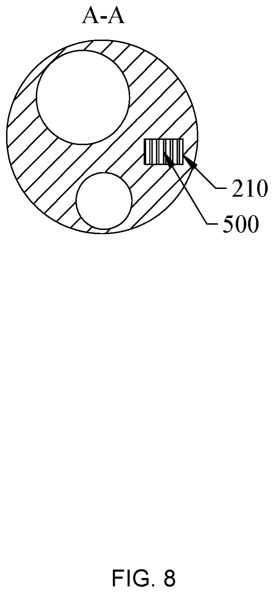

FIG. 7 is a cross-sectional view of a distal end of an endoscopic cutting device according to some other embodiments of the present disclosure. FIG. 8 is a schematic diagram illustrating a cross-section along a line A-A of the endoscopic cutting device according to some embodiments of FIG. 7.

In some embodiments, the first limiting member 500 limits both an axial displacement of the bending segment 320 relative to the first lumen 210 and a circumferential displacement of the bending segment 320 relative to the first lumen 210.

As shown in FIG. 7 and FIG. 8, in some embodiments, at least a portion of the first limiting member 500 has a non-circular cross-sectional shape such that the first limiting member 500 forms a circumferential limitation with the first lumen 210. For example, the cross-sectional shape of the first limiting member 500 includes, but is not limited to, a triangle, a rectangle, a pentagon, a cogwheel shape, etc.

Exemplarily, the first limiting member 500 includes the axially limiting engagement structure 510 and the circumferentially limiting non-circular cross-sectional portion, then the first limiting member 500 limits both the axial displacement of the cutting wire 300 relative to the first lumen 210 and the circumferential displacement of the cutting wire 300 relative to the first lumen 210. For example, the first limiting member 500 is constructed as a square tower like structure, etc.

In some embodiments, the first limiting member 500 is fixed both axially and circumferentially to the first lumen 210, and, the distal end of the cutting wire 300 is fixed to the first limiting member 500, and the bending segment 320 includes a portion from a position where the distal end of the cutting segment 310 enters the sheath tube 200 to a proximal end of the first limiting member 500, and the rotation segment 330 includes a portion fixed inside the first limiting member 500. In the surgical operation, when the operating portion 100 pulls the cutting wire 300 from the distal end to the proximal end, the cutting wire 300 drives the distal end of the sheath tube 200 to bend through the bending segment 320; when the operating portion 100 drives the cutting wire 300 to rotate, the cutting wire 300 transmits a torque to the first limiting member 500, and the first limiting member 500 transmits the torque to the sheath tube 200 and drives the sheath tube 200 to rotate.

In some embodiments, the first limiting member 500 is constructed as an equal cross-section column in an axial direction of the first limiting member 500, and the first limiting member 500 is fixed within the first lumen 210 through an interference fit, which enables the first limiting member 500 to simultaneously limit the axial displacement and the circumferential displacement of the cutting wire 300 within the first lumen 210. The equal cross-section column refers to a column whose shape and size of an arbitrary cross-section along the axial direction of the first limiting member 500 remain constant. Exemplarily, the first limiting member 500 includes, but is not limited to, a cylinder, a triangular prism, a quadrangular prism, a pentagonal prism, etc., and the column structure is simple to process and saves costs.

According to the above-described technical solution, the axial displacement and the circumferential displacement of the cutting wire 300 relative to the first lumen 210 are limited by the first limiting member 500, so that the bending segment 320 and the rotation segment 330 are set adjacent to each other, to simplify an overall structure.

In the following, some exemplary embodiments of the rotation segment 330 and the associated components (e.g., a second limiting member 600 and the second lumen 240) are described in detail in connection with the accompanying drawings.

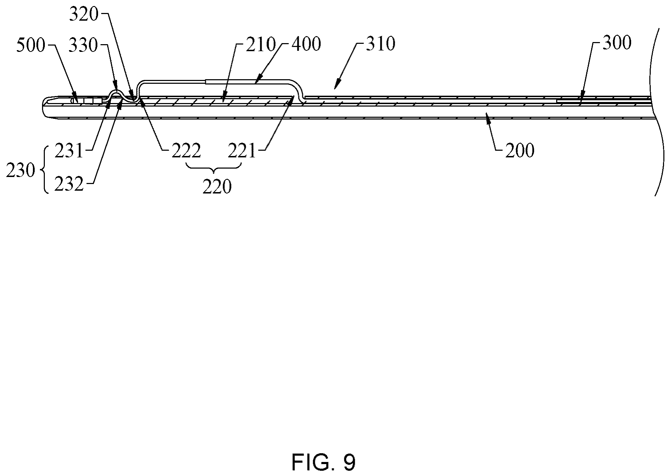

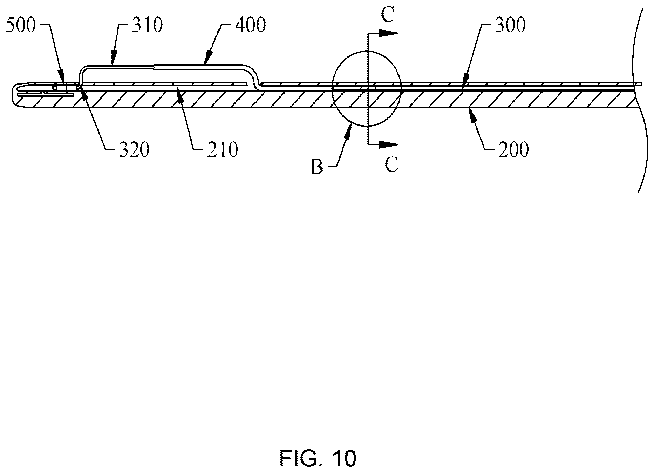

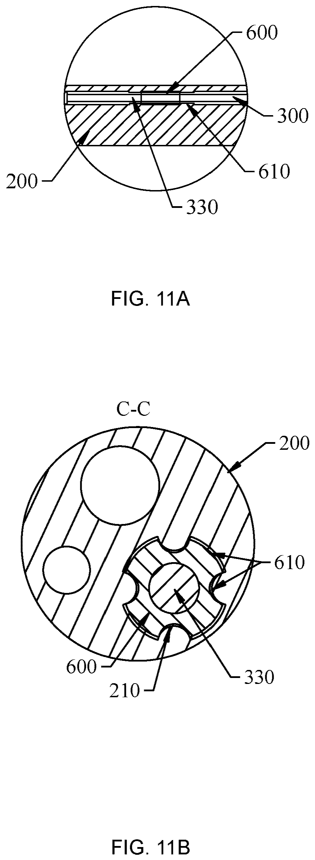

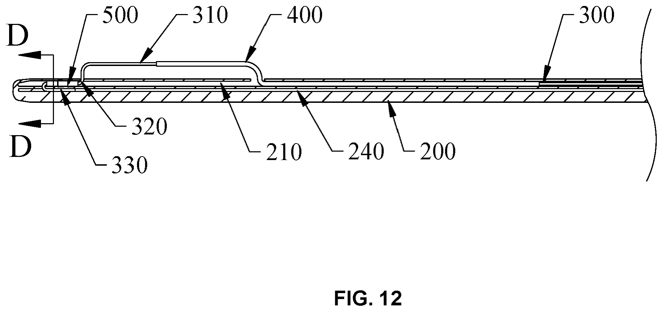

In some embodiments, the rotation segment 330 forms a circumferential limitation with the first lumen 210 so that the rotation segment 330 drives the sheath tube 200 to rotate around the axis of the proximal end of the cutting wire 300 under an effect of a torsion force transmitted from the proximal end to the distal end of the operating portion 100. The axis of the proximal end of the cutting wire 300 refers to an axis of a portion of the cutting wire 300 located on the proximal side of the cutting segment 310. In some embodiments, the rotation segment 330 forms the circumferential limitation with the first lumen 210 by being threaded into a second perforation group 230 (as shown in FIG. 9 and the related description below) for a plurality of times. In some embodiments, the rotation segment 330 forms the circumferential limitation with the first lumen 210 through a second limiting member 600 (described below in FIGs. 10, 11A, 11B and the related descriptions) disposed in the first lumen 210. In some embodiments, the rotation segment 330 forms a circumferential limitation with the first lumen 210 by being disposed within the second lumen 240 (as shown in FIGs. 12-14 and related descriptions below).

FIG. 9 is a cross-sectional view of the distal end of the endoscopic cutting device according to some other embodiments of the present disclosure.

In some embodiments, a sidewall of the first lumen 210 is formed with a first perforation group 220 and at least one second perforation group 230 that are connected to an outer wall of the sheath tube 200, and the at least one second perforation group 230 is disposed on a distal side of the first perforation group 220. The perforation group includes a hole that allows the cutting wire 300 to be threaded out of the sheath tube 200 from an interior of the first lumen 210 and a hole that allows the cutting wire 300 to be threaded into the first lumen 210 from outside of the sheath tube 200.

In some embodiments, the first perforation group 220 includes a first hole 221 and a second hole 222, and the cutting wire 300 is threaded out of the first hole 221 to the outside of the sheath tube 200 to form the cutting segment 310. The cutting segment 310 includes a segment between a position of the cutting wire 300 at the first hole 221 to a position of the cutting wire 300 at the second hole 222.

In some embodiments, the bending segment 320 is threaded from the second hole 222 to the first lumen 210. The bending segment 320 includes a segment between the position of the cutting wire 300 at the second hole 222 and a position where the cutting wire 300 is first threaded into the second perforation group 230.

In some embodiments, the rotation segment 330 is fixed to the first lumen 210 after passing through the second perforation group 230 sequentially. The rotation segment 330 includes a segment between the position where the cutting wire 300 is first threaded into the second perforation group 230 and a position where the cutting wire 300 is finally threaded through the second perforation group 230.

In some embodiments, the sidewall of the first lumen 210 is formed with one or more second perforation groups 230, each second perforation group 230 including an outlet hole 231 and an inlet hole 232. The rotation segment 330 within the first lumen 210 threads through the outlet hole 231 to the outside of sheath tube 200, threads into the first lumen 210 through the inlet hole 232 from outside of the sheath tube 200, and continues to sequentially thread through the other perforation groups. The rotation segment 330 forms a circumferential limitation with the first lumen 210 through the second perforation groups 230, and the rotation segment 330 drives the sheath tube 200 to rotate when the operating portion 100 drives the cutting wire 300 to rotate.

In some embodiments, a plurality of second perforation groups 230 are sequentially arranged along an axial direction of the sheath tube 200. In some embodiments, the plurality of second perforation groups 230 are randomly arranged in the axial direction and a circumferential direction of the sheath tube 200. The present disclosure does not limit the arrangement of the plurality of second perforation groups 230.

According to the solution in the above embodiment, by arranging the one or more second perforation groups 230 to cooperate with the rotation segment, a simple structure is achieved, and a rotation stress and a bending stress are dispersed, so that a risk for the cutting wire 300 to disengage is reduced.

FIG. 10 is a cross-sectional view illustrating a distal end of an endoscopic cutting device according to some other embodiments of the present disclosure. FIG. 11A is a partially enlarged view illustrating a region B of the endoscopic cutting device according to some embodiments of FIG. 10. FIG. 11B is a schematic diagram illustrating a cross-section along a line C-C of the endoscopic cutting device according to some embodiments of FIG. 10.

As shown in FIG. 10 and FIG. 11A, in some embodiments, the rotation segment 330 is disposed within the first lumen 210. In some embodiments, the rotation segment 330 is disposed on a proximal side of the cutting segment 310, or, the rotation segment 330 is disposed between the bending segment 320 and the first limiting member 500.

In some embodiments, the endoscopic cutting device further includes the second limiting member 600. The second limiting member 600 is axially movably disposed within the first lumen 210, at least a portion of the rotation segment 330 is fixed to the second limiting member 600, and the second limiting member 600 is configured to limit a circumferential displacement of the rotation segment 330 relative to the first lumen 210.

Exemplarily, the portion of the cutting wire 300 threaded into the second limiting member 600 constitutes the rotation segment 330, and when the operating portion 100 controls the cutting wire 300 to rotate, the rotation segment 330 drives the second limiting member 600 to rotate, and the second limiting member 600 drives the sheath tube 200 to rotate. Exemplarily, the rotation segment 330 is integrally molded with the second limiting member 600 or fixed to the second limiting member 600 by snap-fitting, bonding, etc.

As shown in FIG. 11B, in some embodiments, a circumferentially limiting structure 610 is formed between the second limiting member 600 and an inner wall of the first lumen 210. The limiting structure 610 includes at least one concave portion and at least one convex portion extending in an axial direction, the at least one concave portion is disposed on one of the outer wall of the second limiting member 600 or the inner wall of the first lumen 210, the at least one convex portion is disposed on the other of the outer wall of the second limiting member 600 and the inner wall of the first lumen 210, and the at least one concave portion and the at least one convex portion cooperate with each other to limit the circumferential displacement between the limiting structure 610 and the inner wall of the first lumen 210.

In some embodiments, the cross-section of the second limiting member 600 constitutes a symmetrical structure in a form of a crosshatch pattern, and the inner wall of the first lumen 210 includes the convex portion that cooperates with the second limiting member 600, and when the cutting wire 300 drives the second limiting member 600 to rotate, a force applied by the second limiting member 600 on a sidewall of the first lumen 210 is more uniform in the circumferential direction, thereby avoiding a failure of torque applied by the second limiting member 600 on the sheath tube 200.

In some embodiments, a length of the concave portion and/or the convex portion of the inner wall of the first lumen 210 in the axial direction is greater than a length of the second limiting member 600 in the axial direction to ensure that the second limiting member 600 always maintains a cooperated state with the concave portion and/or the convex portion of the inner wall of the first lumen 210 when moving axially. In some embodiments, the length of the concave portion and/or the convex portion of the inner wall of the first lumen 210 in the axial direction is 5 to 20 times the length of the second limiting member 600 in the axial direction. In some embodiments, the concave portions and/or the convex portions are all disposed on the inner wall of the first lumen 210 along the axial direction to simplify a processing of the first lumen 210.

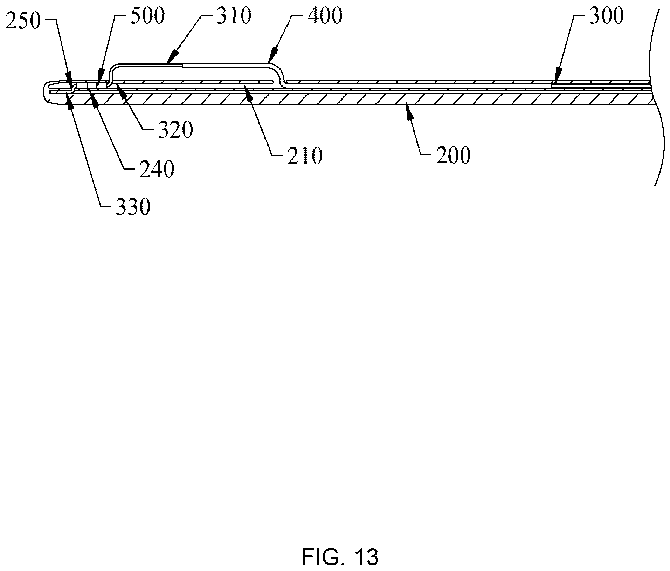

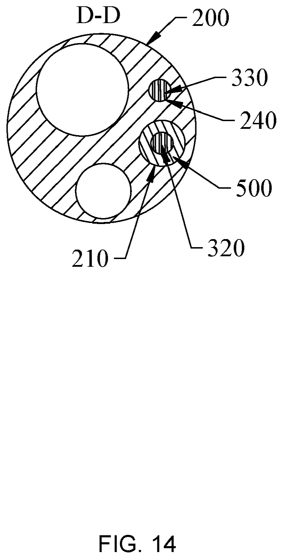

FIG. 12 is a cross-sectional view of a distal end of an endoscopic cutting device according to some other variant embodiments of the present disclosure. FIG. 13 is a cross-sectional view illustrating a distal end of an endoscopic cutting device according to some other variant embodiments of the present disclosure. FIG. 14 is a schematic diagram illustrating a cross-section along a line D-D of the endoscopic cutting device according to some embodiments of FIG. 12.

As shown in FIG. 3, and FIGs. 12-14, in some embodiments, the cutting wire lumen further includes a second lumen 240 communicating with the first lumen 210, the second lumen 240 is disposed non-collinearly to the first lumen 210, and the rotation segment 330 is disposed within the second lumen 240. The "disposed non-collinearly" may be that the first lumen 210 and the second lumen 240 do not lie on the same axis, for example, the first lumen 210 and the second lumen 240 may be parallel or intersect at an angle. When the operating portion 100 rotates the cutting wire 300, the rotation segment 330 disposed in the second lumen 240 may generate a torque on the sheath tube 200 to rotate the sheath tube 200. As the second lumen 240 is disposed non-collinearly with the first lumen 210, a force arm of the rotation segment 330 that applies a force on the sheath tube 200 grows, thereby increasing the torque to make the sheath tube 200 rotate more easily.

In some embodiments, the second lumen 240 is parallel to the first lumen 210 or intersects with the first lumen 210 at an angle. For example, the second lumen 240 is perpendicular to the first lumen 210, or the angle between the second lumen 240 and the first lumen 210 is 30°, 45°, 60°, etc.

In some embodiments, a length of the second lumen 240 is set according to actual needs. For example, the length of the second lumen 240 is equal to a length of the rotation segment 330. As another example, the length of the second lumen 240 is greater than the length of the rotation segment 330 and less than an axial length of the sheath tube 200. As another example, the length of the second lumen 240 is equal to the axial length of the sheath tube 200.

In some embodiments, the second lumen 240 is parallel to the first lumen 210, the rotation segment 330 enters the second lumen 240 and extends either toward the distal end of the sheath tube 200 (as shown in FIG. 13) or toward a proximal end of the sheath tube 200 (as shown in FIG. 12).

In some embodiments, a sidewall of the second lumen 240 is formed with a via 250 communicating the first lumen 210, and the cutting wire 300 passes through the via 250 within the first lumen 210 and enters the second lumen 240. In some embodiments, the via 250 is disposed at an end portion of the second lumen 240. In some embodiments, the via 250 is disposed between two ends of the second lumen 240.

In some embodiments, an inner diameter of the second lumen 240 is equal to an outer diameter of the rotation segment 330, so that the rotation segment 330 is fitted to the inner wall of the second lumen 240, thereby applying a rotational force of the rotation segment 330 to the sheath tube 200, and improving an efficiency of torque transmission.

In some embodiments, the second lumen 240 is a linear lumen. In some embodiments, the second lumen 240 is a curved lumen.

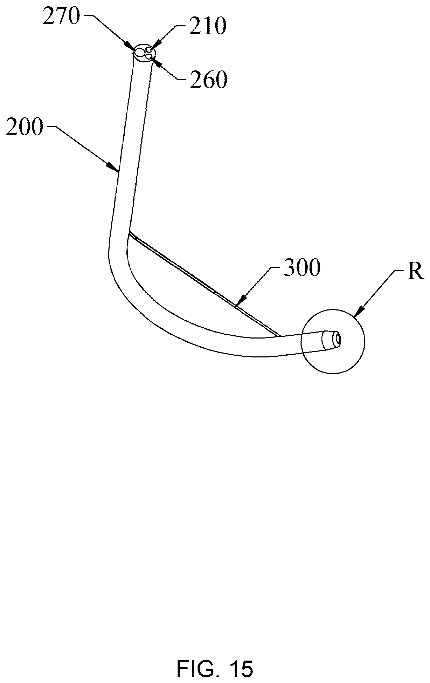

In some embodiments, the second lumen 240 within the sheath tube 200 is replaced by an injection lumen 260 to reduce a count of lumens within the sheath tube 200, and to reduce a cross-sectional size of the sheath tube 200. The following will describe in detail an exemplary embodiment of the endoscopic cutting device provided in Embodiment II of the present disclosure with reference to FIGs. 15-20B. The endoscopic cutting device of Embodiment II is intended to utilize the injection lumen 260 to accommodate the distal end of the cutting wire to reduce the count of lumens within the sheath tube 200. It should be appreciated that the endoscopic cutting device of Embodiment II is suitably combined, modified, or borrowed from the endoscopic cutting device of Embodiment I, without conflict.

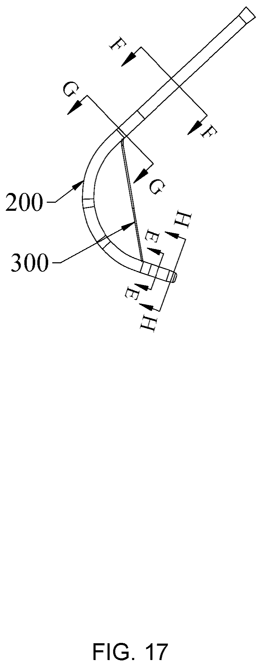

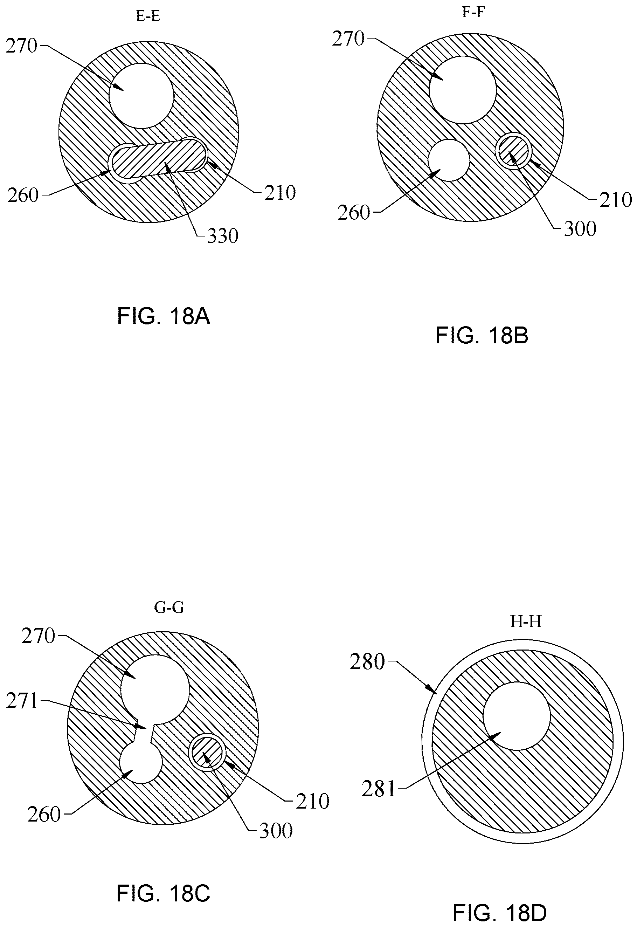

FIG. 15 is a schematic diagram illustrating a structure of a distal end of an endoscopic cutting device according to some other embodiments of the present disclosure; and FIG. 16 is a partial cross-sectional view of the distal end of the endoscopic cutting device according to FIG. 15. FIG. 17 is a side view of the distal end of the endoscopic cutting device according to FIG. 15. FIGs. 18A-18D are schematic diagrams illustrating cross-sections at different positions of the endoscopic cutting device according to FIG. 17

The endoscopic cutting device provided in Embodiment II of the present disclosure includes the sheath tube 200 and the cutting wire 300 disposed within the sheath tube 200, as shown in FIGs. 15-18D.

In some embodiments, the sheath tube 200 includes the injection lumen 260 and the first lumen 210 for accommodating the cutting wire 300. A first communication hole 261 is formed between the injection lumen 260 and the first lumen 210, at least a portion of the cutting wire 300 passes through the first communication hole 261 and is fixed into the injection lumen 260. For example, the rotation segment 330 bends from the first communication hole 261 into the injection lumen 260. By utilizing the injection lumen 260 to accommodate the cutting wire 300, a situation where too many lumens are opened so as to reduce a strength of the distal end of the sheath tube 200 is avoided (e.g., no need to configure a second lumen), thereby preventing an unintended bending deformation of the sheath tube 200.

Exemplarily, the first communication hole 261 is disposed at a distal end of the injection lumen 260, the distal end of the cutting wire 300 constitutes the rotation segment 330, and the rotation segment 330 is bent at a distal end of the first lumen 210 and enters the injection lumen 260 through the first communication hole 261. When the operating portion 100 rotates the cutting wire 300, the rotation segment 330 disposed in the first lumen 210 and the injection lumen 260 may generate a torque on the sheath tube 200, causing the sheath tube 200 to rotate. With at least a portion of the rotation segment 330 disposed within the injection lumen 260, there is no need to additionally increase the count of lumens, which reduces a processing difficulty, and is conducive to reducing a cross-sectional size of the sheath tube 200. In addition, the rotation segment 330 is arranged separately from the bending segment 320, which avoids a stress concentration and prevents the sheath tube 300 from breaking.

In some embodiments, the rotation segment 330 extends toward the distal end of the sheath tube 200 to facilitate assembly. As shown in FIG. 18A, in some embodiments, the rotation segment 330 extends toward a proximal end of the sheath tube 200, i.e., the rotation segment 330 extends distally within the first lumen 210 to the first communication hole 261, and reversely bends into the injection lumen 260. An overall structure of the rotation segment 330 is a hook, which is able to hook the first communication hole 261 to form an axial limitation and improve a connection stability of the cutting wire 300.

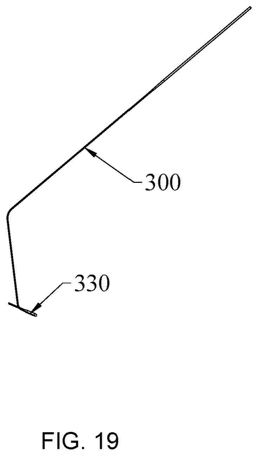

FIG. 19 is a schematic diagram illustrating a structure of a cutting wire 300 according to some other embodiments of the present disclosure.

As shown in FIGs. 16-19, in some embodiments, a diameter of the rotation segment 330 is greater than or equal to an inner diameter of the injection lumen 260. In this way, the rotation segment 330 forms an interference fit with the injection lumen 260, thereby limiting an axial displacement of the rotation segment 330 relative to the injection lumen 260, and improving a connection stability of the cutting wire 300. Exemplarily, the diameter of the rotation segment 330 is in a range of 0.2 mm-0.6 mm, and preferably, the diameter of the rotation segment 330 is 0.45 mm.

In some embodiments, the length of the rotation segment 330 within the injection lumen 260 is in a range of 1 mm-15 mm. Exemplarily, a length of the distal end of the cutting wire 300 is about 5 mm, and a length of the cutting wire 300 bending into the injection lumen 260 is about 2 mm, the range of length facilitates an assembly while balancing a cooperation stability between the cutting wire 300 and the injection lumen 260.

In some embodiments, the diameter of the cutting segment 310 of the cutting wire 300 is smaller than diameters of the other portions of the cutting wire 300, making the cutting segment 310 sharper and have a higher cutting precision. Exemplarily, the cutting wire 300 with a diameter of 0.45 mm is selected, and the diameter of the cutting segment 310 is about 0.24 mm. A processing manner of the cutting wire 300 includes: wrapping the cutting wire 300 with a diameter of 0.45 mm to expose only the cutting segment 310, and the wrapped cutting wire 300 is immersed in a dissolving liquid to make the diameter of the cutting segment 310 thinner, for example, to dissolve to 0.24 mm, and finally, the processed cutting wire is assembled to the sheath tube. The cutting segment 310 is thinner compared to segments such the bending segment 320, the rotation segment 330, and a portion at the proximal end, so as to ensure a superior cutting performance.

In some embodiments, the rotation segment 330 is disposed with a developing structure (not shown in the figures), and the developing structure may indicate a position of the rotation segment 330, which facilitates guiding an operator to bend the rotation segment 330 from the first lumen 210 into the injection lumen 260.

As shown in FIG. 16, Embodiment II of the present disclosure also provides other embodiments of the endoscopic cutting device. In some embodiments, the sheath tube 200 includes the guide wire lumen 270 and the injection lumen 260, with the second communication hole 271 formed between the guide wire lumen 270 and the injection lumen 260. For example, the second communication hole 271 is disposed between a bending origin of the sheath tube 200 and the middle portion of the sheath tube 200 for directing a solution from the injection lumen 260 to the guide wire lumen 270, where the bending origin is a proximal endpoint of a curved segment of the sheath tube 200. The curved segment refers to a portion corresponding to a curved portion of the sheath tube 200 between the two ends of the cutting segment of the cutting wire after the cutting wire pulls the sheath tube 200 to bend. The "middle portion of the sheath tube 200" is based on an entire length of the sheath tube 200. In the axial direction of the sheath tube 200, segments that is at a certain distance to the left or right of the midpoint of the sheath tube 200 may be referred to as the middle portion of the sheath tube 200. By disposing the second communication hole 271 to communicate the guide wire lumen 270 and the injection lumen 260, and to guide the solution of the injection lumen to the guide wire lumen 270, a setting position at the distal end of the sheath tube 200 is reserved for other structures at the distal end, thereby making a product structure more reasonable and stable. In one embodiment, the distal end portion of the injection lumen 260 located at second communication hole 271 is used as a lumen for installing the cutting wire 300, so as to reduce the count of lumens, avoid opening too many lumens, and improve a structural strength of the distal end of the sheath tube; further, the second communication hole 271 is set between the bending origin of the curved segment and the middle portion of the sheath tube, so as to avoid the curved segment and ensure the structural strength of the distal end of the sheath tube.

In some embodiments, the second communication hole 271 is disposed at the bending origin, i.e., a position where the cutting segment extends out of the sidewall of the sheath tube 200 from the proximal end to the distal end, to facilitate processing. In some embodiments, after the cutting segment 310 forms a knife portion, the corresponding portion of the sheath tube 200 bends to form the curved segment. To avoid the second communication hole 271 from affecting the curved shape or the strength of the sheath tube 200, preferably, the position of the second communication hole 271 is set to avoid the bending portion of the sheath tube 200, and disposed at a position closer to the middle portion of the sheath tube 200 on the proximal side of the bending portion of the sheath tube 200. In other words, the second communication hole is disposed between the middle portion of the sheath tube 200 and the position where the cutting segment 310 extends out of the sheath tube 200, so as to ensure the structural strength of the bending portion of the sheath tube 200.

In some embodiments, the second communication hole 271 and the first communication hole 261 are axially offset along the sheath tube 200. Exemplarily, the second communication hole 271 is located between a middle portion of the sheath tube 200 and the first communication hole 261, i.e., the second communication hole 271 is disposed within a curved segment of the sheath tube 200, or is disposed on other segments of a proximal end of the curved segment.

In some embodiments, a cross-sectional area of the second communication hole 271 is greater than a cross-sectional area of the injection lumen 260, thereby avoiding a situation of excessive pressure or clogging of the solution from the injection lumen 260 into the guide wire lumen 270.

In some embodiments, a sealing member 272 is disposed in the injection lumen 260, the sealing member 272 is located between the first communication hole 261 and the second communication hole 271 for sealing the injection lumen 260.

As shown in FIG. 18B, on the proximal side of the second communication hole 271, the guide wire lumen 270 and the injection lumen 260 are independent from each other. As shown in FIG. 18C, at the second communication hole 271, the guide wire lumen 270 and the injection lumen 260 are communicated with each other. Exemplarily, after the guide wire lumen 270 and the injection lumen 260 are communicated, the injection lumen 260 is sealed by the sealing member 272, and in use, the solution (e.g., the developer) enters from the proximal end of the injection lumen 260, flows through the second communication hole 271 into the guidewire lumen 270, and flows along the guide wire lumen 270 to the distal end of the sheath tube 200. In this way, after the distal end portion of the injection lumen 260 is configured to accommodate the rotation segment 330, the distal end portion of the guide wire lumen 270 is configured to direct a flow of solution, and without increasing the count of lumens originally in the sheath tube 200, an arrangement position is provided for the rotation segment 330 without affecting various functions of the sheath tube 200.

In some embodiments, the sealing member 272 includes, but is not limited to, a sealant and a rubber elastomer block. In some other embodiments, the injection lumen 260 is also sealed by melting, etc.

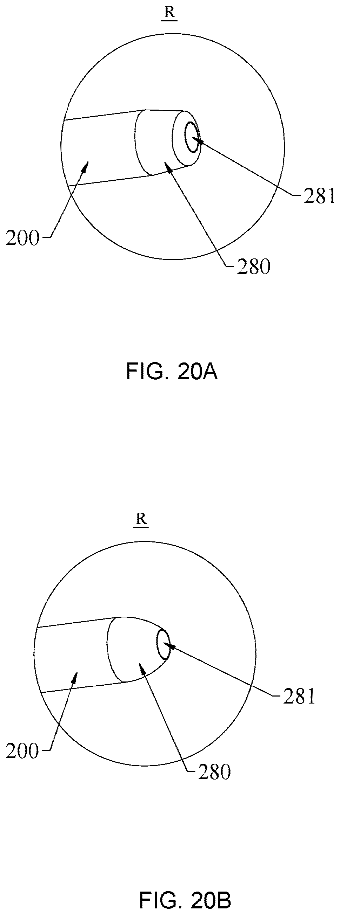

FIG. 20A is a partially enlarged view of a region R at the distal end of the endoscopic cutting device according to FIG. 15. FIG. 20B is a localized enlarged view of a region R at the distal end of a variant embodiment of the endoscopic cutting device according to FIG. 15.

In some embodiments, the sheath tube 200 includes a tip structure 280 fixed to a distal end of the sheath tube 200, a diameter of a proximal end of the tip structure 280 is equal to a diameter of the distal end of the sheath tube 200, and the diameter of the tip structure 280 decreases from a proximal end to a distal end. Exemplarily, the tip structure 280 is constructed in a shape of a frustum of a cone (as shown in FIG. 20A) or a hemisphere (as shown in FIG. 20B) with a great proximal cross-section and a small distal cross-section. By disposing the tip structure 280, a size of the distal end of the sheath tube 200 is decreased, and the sheath tube 200 has a guiding effect when traveling.

Combining FIG. 16 and FIG. 18D, in some embodiments, the tip structure 280 includes a distal outlet lumen 281, and a proximal end of the distal outlet lumen 281 is connected to the distal end of the guide wire lumen 270, and both a guide wire and the solution are guided from the distal outlet lumen 281. In some embodiments, a diameter of the distal outlet lumen 281 decreases progressively from the proximal end to the distal end, which facilitates further size reduction of the tip structure 280. In some embodiments, the diameter of the distal outlet lumen 281 remains constant to facilitate processing.

In some embodiments, an axial length of the tip structure 280 is in a range of 1 mm-10 mm. In this range of length, the tip structure 280 is guided without damaging a tissue.

In the following, some exemplary embodiments of the operating portion 100 are described in detail again combining FIG. 1.

As shown in FIG. 1, in some embodiments, the operating portion 100 includes a pulling portion 140 and a rotating portion 150. Exemplarily, the pulling portion 140 is configured to control the cutting wire 300 to move from the distal end to the proximal end, so that the bending segment 320 of the cutting wire 300 drives the distal end of the sheath tube 200 to bend, and when the distal end of the sheath tube 200 is bent, a portion of the cutting wire 300 disposed on an outer side of the sheath tube 200 constitutes a knife portion, which facilitates cutting of a body tissue (e.g., the nipple sphincter). Exemplarily, the rotating portion 150 is configured to control the cutting wire 300 to rotate, so that the rotation segment 330 of the cutting wire 300 drives the sheath tube 200 to rotate, thereby facilitating an operator to orient the aforementioned knife portion towards an orientation to be cut (e.g., towards an 11 o'clock direction of the nipple sphincter). By separately disposing the pulling portion 140 and the rotating portion 150, the rotating operation and the axial moving operation of the cutting wire 300 are separately controlled to avoid misoperation.

In some embodiments, the rotating portion 150 is disposed adjacent to the pulling portion 140 on the operating portion 100, e.g., the rotating portion 150 is connected to a proximal end of the pulling portion 140, which is convenient for the operator to control. In some embodiments, the rotating portion 150 is spaced apart from the pulling portion 140, e.g., the pulling portion 140 is disposed at the proximal end of the operating portion 100, and the rotating portion 150 is disposed on the liner assembly frame 110.

In some embodiments, the pulling portion 140 includes at least one slider, which is slidably disposed on the operating portion 100, and the proximal end of the cutting wire 300 is fixed to the slider. When the operator pulls the slider, the slider drives the cutting wire 300 to move axially within the sheath tube 200.

In some embodiments, the rotating portion 150 includes a rotating bracelet, which is rotatably disposed at the proximal end of the operating portion 100, and is fixedly connected to the pulling portion 140. When the operator controls the rotating bracelet to rotate, the rotating bracelet drives the pulling portion 140 to rotate together, and the pulling portion 140 drives the cutting wire 300 to rotate. In some embodiments, the rotating portion 150 includes a hand wheel (not shown in the figures). The hand wheel is disposed on the liner assembly frame 110, and the proximal end of the cutting wire 300 passes through the hand wheel and cooperates with the hand wheel, so that when the operator controls the hand wheel to rotate, the hand wheel may drive the cutting wire 300 to rotate around its own axis.

Some embodiments of the present disclosure further provide an endoscope including the endoscopic cutting device as in any of the above embodiments.

Possible beneficial effects of embodiments of the present disclosure include, but are not limited to:
(1) The bending segment and the rotation segment of the cutting wire are disposed separately, so that the force received by the cutting wire in controlling the bending of the sheath tube and the force received by the cutting wire in controlling the rotation of the sheath tube may be dispersed in different positions of the cutting wire, thus avoiding an over-concentration of the stress of the cutting wire in the bending and rotation of the control sheath tube and preventing breakage or disengagement of the cutting wire.
(2) The axial displacement of the bending segment is limited by the first limiting member, but the circumferential displacement of the bending segment is not limited by the first limiting member, so that the axial displacement and the circumferential displacement are dispersed out to prevent the cutting wire from breakage and disengagement.
(3) The axial displacement and the circumferential displacement of the cutting wire relative to the first lumen are limited by the first limiting member, so that the bending segment and the rotation segment are disposed adjacent to each other to simplify the overall structure.
(4) By providing the second perforation group to match the rotation segment, the structure is simple, and the rotation stress and the bending stress are dispersed to reduce a risk for the cutting wire to disengage.
(5) By providing the second lumen and arranging the second lumen non-collinearly with the first lumen, the force arm of the force applied by the rotation segment to the sheath tube increases, thereby increasing the torque and making it easier to drive the sheath tube to rotate.
(6) At least a portion of the rotation segment is arranged in the injection lumen, thereby avoiding adding additional lumens, reducing the difficulty of processing, and contributing to a reduction of the cross-sectional size of the sheath tube.
(7) By providing the first communication hole to communicate the injection lumen and the cutting wire lumen, the rotation segment of the cutting wire is disposed within the injection lumen to avoid opening too many lumens (e.g., without the need to configure a second lumen) to reduce the strength of the distal end of the sheath tube, thereby preventing the sheath tube from unintended bending deformations.
(8) By disposing the second communication hole to communicate the guide wire lumen and the injection lumen, the setting position is reserved at the distal end of the sheath tube, thereby reserving the setting space for other structures at the distal end to make the structure of the product reasonable and stable; and the second communication hole is set between the bending origin and the middle portion of the sheath tube, which is able to avoid the curved segment and ensure the structural strength of the distal end of the sheath tube.

It is noted that different embodiments may generate different beneficial effects, and in different embodiments, the beneficial effects generated may be any one or more of the above, or may be other possibly beneficial effects.

The basic concepts have been described above, and it is clear that to those skilled in the art, the above-detailed disclosure serves only as an example and does not constitute a limitation of the present disclosure. While not expressly stated herein, those skilled in the art may make various modifications, improvements, and amendments to the present disclosure. Such modifications, improvements, and amendments are suggested in the present disclosure, so such modifications, improvements, and amendments remain within the spirit and scope of the exemplary embodiments of the present disclosure.

Also, the present disclosure uses specific words to describe the embodiments of the present disclosure. Such as "an embodiment," "one embodiment," and/or "some embodiments" means a feature, structure, or characteristic associated with at least one embodiment of the present disclosure. Accordingly, it should be emphasized and noted that "an embodiment," "one embodiment" or "an alternative embodiment" in different places in the present disclosure do not necessarily refer to the same embodiment. In addition, certain features, structures, or characteristics in one or more embodiments of the present disclosure may be suitably combined.

Similarly, it should be noted that in order to simplify the presentation of the disclosure of the present disclosure, and thereby aiding in the understanding of one or more embodiments, the foregoing descriptions of embodiments of the present disclosure sometimes combine a variety of features into a single embodiment, accompanying drawings, or a description thereof. However, this manner of disclosure does not imply that more features are required for the objects of the present disclosure than are mentioned in the claims. Rather, claimed subject matter may lie in less than all features of a single foregoing disclosed embodiment.

Counts describing the count of components, attributes, and properties are used in some embodiments, and it is to be understood that such numbers used in the description of embodiments are modified in some examples by the modifiers "about," "approximately," or "substantially". Unless otherwise noted, the terms "about," "approximately," or "substantially" indicates that a ±20% variation in the stated count is allowed. Correspondingly, in some embodiments, the numerical parameters used in the present disclosure and the claims are approximations, which are subject to change depending on the desired characteristics of individual embodiments. In some embodiments, the numerical parameters should take into account the specified count of valid digits and employ general place-keeping. While the numerical domains and parameters used to confirm the breadth of their ranges in some embodiments of the present disclosure are approximations, in specific embodiments such values are set to be as precise as practicable.

Finally, it should be understood that the embodiments described herein are used only to illustrate the principles of the embodiments of the present disclosure. Other deformations may also fall within the scope of this manual. As such, alternative configurations of embodiments of the present disclosure may be viewed as consistent with the teachings of the present disclosure as an example, not as a limitation. Correspondingly, the embodiments of the present disclosure are not limited to the embodiments expressly presented and described herein.

## Claims

1. An endoscopic cutting device comprising:
an operating portion;
a sheath tube including an injection lumen and a cutting wire lumen, a proximal end of the sheath tube being connected to the operating portion;
a cutting wire at least partially disposed within the cutting wire lumen, a proximal end of the cutting wire being connected to the operating portion, and the cutting wire including a cutting segment, a limiting portion, and a rotation segment disposed at different positions of the cutting wire, wherein the cutting segment extends out of the sheath tube from a sidewall of the sheath tube, the limiting portion is disposed within the cutting wire lumen and is configured to form an axial limitation with the cutting wire lumen, and the rotation segment is disposed either in the injection lumen or in the cutting wire lumen and is configured to drive the sheath tube to rotate around an axis of the proximal end of the cutting wire when the operating portion rotates the cutting wire.

2. The endoscopic cutting device of claim 1, wherein the cutting wire lumen includes a first lumen, and the limiting portion includes a bending segment, the bending segment is disposed on a distal side of the cutting segment, at least a portion of the bending segment is disposed in the first lumen, and the bending segment is configured to drive a distal end of the sheath tube to bend when the operating portion pulls the cutting wire from a distal end to a proximal end of the endoscopic cutting device.

3. The endoscopic cutting device of claim 2, wherein the limiting portion further includes a first limiting member disposed within the first lumen, the bending segment is fixed to the first limiting member, and the first limiting member is configured to limit an axial displacement of the bending segment relative to the first lumen;

4. The endoscopic cutting device of claim 3, wherein an engagement structure with an axial limitation is formed between the first limiting member and an inner wall of the first lumen, and the engagement structure includes at least one limiting recess and at least one limiting protrusion extending along a circumferential direction, the at least one limiting recess is disposed on one of an outer wall of the first limiting member and the inner wall of the first lumen, the at least one limiting protrusion is disposed on the other of the outer wall of the first limiting member and the inner wall of the first lumen, and the at least one limiting recess and the at least one limiting protrusion are mutually engaged.

5. The endoscopic cutting device of claim 4, wherein a shape of a cross-sectional of the first limiting member is circular, and the engagement structure includes one or more of a tower-like structure, a serrated structure, and a threaded structure.

6. The endoscopic cutting device of claim 3, wherein at least a portion of a cross-sectional shape of the first limiting member is non-circular, and the first limiting member forms a circumferential limitation with the first lumen.

7. The endoscopic cutting device of claim 3, wherein the first limiting member is constructed as an isotropic cross-sectioned column along an axial direction of the first limiting member, and the first limiting member is fixed in the first lumen through an interference fit.

8. The endoscopic cutting device of claim 2, wherein a sidewall of the first lumen is formed with a first perforation group and at least one second perforation group communicating with an outer wall of the sheath tube, wherein the at least one second perforation group is disposed at a distal side of the first perforation group, the first perforation group includes a first hole and a second hole, the cutting wire penetrates from the first hole to an outer side of the sheath tube to form the cutting segment, the bending segment penetrates from the second hole to the first lumen, and the rotation segment sequentially penetrates though from the at least one second perforation group sequentially and is fixed in the first lumen.

9. The endoscopic cutting device of claim 2, further comprising a second limiting member axially movably disposed within the first lumen, at least a portion of the rotation segment is fixed to the second limiting member, and the second limiting member is configured to limit a circumferential displacement of the rotation segment relative to the first lumen.

10. The endoscopic cutting device of claim 9, wherein a circumferentially limiting structure is formed between the second limiting member and an inner wall of the first lumen, the circumferentially limiting structure includes at least one concave portion and at least one convex portion extending along an axial direction, the at least one concave portion is disposed on one of the outer wall of the second limiting member or the inner wall of the first lumen, the at least one convex portion is disposed on the other of the outer wall of the second limiting member and the inner wall of the first lumen, and the at least one concave portion and the at least one convex portion cooperate with each other.

11. The endoscopic cutting device of claim 2, wherein the cutting wire lumen further includes a second lumen communicating with the first lumen, the second lumen is disposed non-collinearly to the first lumen, and the rotation segment is disposed within the second lumen.

12. The endoscopic cutting device of claim 11, wherein the second lumen is parallel to the first lumen, the rotation segment extends within the second lumen toward the distal end of the sheath tube or toward the proximal end of the sheath tube.

13. The endoscopic cutting device of claim 11, wherein an inner diameter of the second lumen is equal to an outer diameter of the rotation segment.

14. The endoscopic cutting device of claim 2, wherein a first communication hole is formed between the injection lumen and the first lumen, the first communication hole is located at a distal end of the injection lumen, and the rotation segment bends from the first communication hole into the injection lumen, and extends toward the distal end of the sheath tube or toward the proximal end of the sheath tube.

15. The endoscopic cutting device of claim 14, wherein a diameter of the rotation segment is greater than or equal to an inner diameter of the injection lumen.

16. The endoscopic cutting device of claim 14, wherein the sheath tube further includes a guide wire lumen, a second communication hole is formed between the guide wire lumen and the injection lumen, and the second communication hole is located between a middle of the sheath tube and the first communication hole, a sealing member is disposed between the first communication hole and the second communication hole to seal the injection lumen.

17. The endoscopic cutting device of claim 16, wherein the sheath tube includes a tip structure fixed to the distal end of the sheath tube, a diameter of a proximal end of the tip structure is equal to a diameter of the distal end of the sheath tube, and a diameter of the tip structure decreases progressively from the proximal end to a distal end; and
the tip structure includes a distal outlet lumen whose proximal end is connected to a distal end of the guide wire lumen.

18. The endoscopic cutting device of claim 16, wherein the rotation segment is disposed with a developing structure.

19. The endoscopic cutting device of claim 2, wherein the operating portion includes a pulling portion and a rotating portion, wherein the pulling portion is configured to control the cutting wire to move proximally, the bending segment drives the distal end of the sheath tube to bend, the rotating portion is configured to control the cutting wire to rotate, and the rotation segment drives the sheath tube to rotate; and
the rotating portion is connected to a proximal end of the pulling portion; or
the rotating portion is spaced apart from the pulling portion.

20. The endoscopic cutting device of claim 1, wherein the sheath tube further includes a guide wire lumen, a second communication hole is formed between the guide wire lumen and the injection lumen, and the second communication hole is located between a bending origin of the sheath tube and a middle of the sheath tube to direct a solution in the injection lumen to the guide wire lumen, wherein the bending origin is a proximal endpoint of a curved segment of the sheath tube.

21. The endoscopic cutting device of claim 20, wherein the cutting wire lumen includes a first lumen for accommodating the cutting wire, a first communication hole is formed between the injection lumen and the first lumen, the first communication hole is located at a distal end of the injection lumen, and the rotation segment passes through the first communication hole and is fixed into the injection lumen.

22. The endoscopic cutting device of claim 21, wherein a diameter of the rotation segment is greater than or equal to an inner diameter of the injection lumen.

23. The endoscopic cutting device of claim 20, wherein a cross-sectional area of the second communication hole is greater than a cross-sectional area of the injection lumen.

24. The endoscopic cutting device of claim 20, wherein the injection lumen is disposed with a sealing member disposed between the second communication hole and a distal end of the injection lumen for sealing the injection lumen.

25. The endoscopic cutting device of claim 1, wherein the cutting wire lumen includes a first lumen accommodating the cutting wire, a first communication hole is formed between the injection lumen and the first lumen, and the rotation segment penetrates through the first communication hole and is fixed in the injection lumen.

26. The endoscope cutting device of claim 25, wherein the sheath tube further includes a guide wire lumen, a second communication hole is formed between the guide wire lumen and the injection lumen, and the second communication hole is configured to direct a solution in the injection lumen to the guide wire lumen.

27. The endoscope cutting device of claim 26, wherein the first communication hole is located at a distal end of the injection lumen, and the second communication hole and the first communication hole are axially offset along the axial direction of the sheath tube.

28. The endoscopic cutting device of claim 26, wherein a cross-sectional area of the second communication hole is greater than a cross-sectional area of the injection lumen.

29. The endoscopic cutting device of claim 25, wherein a diameter of the rotation segment is greater than or equal to an inner diameter of the injection lumen.
